# FASCICULE DE BREVET EUROPEEN

(11) **EP 3 716 916 B1**
(45) Date de publication et mention de la délivrance du brevet: **09.02.2022**
(21) Numéro de dépôt: 18833084.9
(22) Date de dépôt: 27.11.2018
(51) Int. Cl.: A61F 2/40

(54) **COMPOSANT HUMÉRAL D'ANCRAGE SANS TIGE POUR IMPLANT HUMÉRAL DE PROTHÈSE D'ÉPAULE**
KOMPONENTE FÜR DIE HUMERALE VERANKERUNG OHNE SCHAFT FÜR DAS HUMERALE IMPLANTAT DER SCHULTERPROTHESE
HUMERAL ANCHOR COMPONENT WITHOUT STEM FOR HUMERAL IMPLANT OF SHOULDER PROSTHESIS

(30) Priorité: 28.11.2017 FR 1761299
(43) Date de publication de la demande: 07.10.2020
(73) Titulaire: Shoulder Friends Institute, 75008 Paris (FR)
(72) Inventeur: LEFEBVRE, Yves, 67000 Strasbourg (FR); AUDEBERT, Stéphane, 59268 Blecourt (FR); BARTH, Johannes, 38240 Meylan (FR); CHAROUSSET, Christophe, 75017 Paris (FR); GARRET, Jérôme, 69760 Limonest (FR); GALLINET, David, 25870 Geneuille (FR); GODENECHE, Arnaud, 69450 Saint Cyr Au Mont D'Or (FR); GUERY, Jacques, 58000 Nevers (FR); JOUDET, Thierry, 33500 Libourne (FR)
(74) Mandataire: Germain Maureau
(86) Numéro de dépôt international: PCT/FR2018/052994
(87) Numéro de publication internationale: WO 2019/106278

(56) Documents cités:
- EP-A1- 2 965 720
- WO-A1-2013/009407
- WO-A1-2015/112307
- US-A1- 2013 018 476

## Description

La présente invention se rapporte à un composant huméral d'ancrage sans tige pour implant huméral de prothèse d'épaule.

Un composant huméral d'ancrage sans tige, également appelé en anglais « stemless humerai component », est un composant prévu pour un ancrage dans les parties épiphysaire proximal et métaphysaire réséquées de l'humérus sans l'emploi d'une tige d'ancrage implantée dans la partie diaphysaire de l'humérus, et plus spécifiquement dans la cavité médullaire de l'humérus.

Pour réaliser de tels composants huméraux d'ancrage sans tige, il est connu des documents FR 2 980 685, FR 2 997 290 et EP 2 474 288 d'employer une pièce d'ancrage conique, cylindrique ou en forme de coupole munie de dents périphériques sur tout son pourtour, et il est également connu du document EP 2 815 726 d'employer une cupule d'ancrage présentant des segments latéraux flexibles séparés par des fentes, certains des segments ayant des ergots saillants et d'autres ayant des encoches en creux.

L'état de la technique peut être également illustré par l'enseignement du document EP 2 830 541 qui décrit un composant huméral d'ancrage sans tige muni d'une bride circulaire, d'un manchon creux et de pattes dentées saillantes en porte-à-faux de la bride circulaire, ainsi que par l'enseignement du document EP 2 663 263 qui divulgue un composant huméral d'ancrage sans tige muni d'une couronne présentant une face inférieure de laquelle fait saillie une pluralité de lames ajourées et acérées.

Il est aussi connu du document EP 2 965 720 un composant huméral d'ancrage sans tige comportant un plot d'ancrage assujetti à quatre ailettes d'ancrage s'étendant radialement en périphérie du plot d'ancrage et écartées angulairement d'un angle de 90 degrés, ces ailettes étant par ailleurs solidaires d'une couronne proximale entourant le plot d'ancrage.

L'état de la technique peut également être illustré par les enseignements des documents US2013/0018476 et WO2013/009407 qui divulgue un composant huméral d'ancrage sans tige comportant un plot d'ancrage assujetti à six ailettes d'ancrage réparties radialement et symétriquement en périphérie du plot d'ancrage.

Tous ces composants huméraux d'ancrage sans tige de l'art antérieur présentent l'inconvénient d'être positionnable essentiellement dans une zone osseuse centrale des parties épiphysaire et métaphysaire réséquées de l'humérus, qui est une zone d'os spongieux où la densité osseuse n'offre pas une stabilité en torsion optimale pour le composant huméral d'ancrage sans tige.

La présente invention a pour but de proposer un composant huméral d'ancrage sans tige qui soit conformé pour offrir une stabilité en torsion accrue.

A cet effet, elle propose un composant huméral d'ancrage sans tige pour implant huméral de prothèse d'épaule, ce composant huméral d'ancrage sans tige comportant un plot d'ancrage assujetti à des ailettes d'ancrage s'étendant radialement en périphérie du plot d'ancrage, où ce composant huméral d'ancrage sans tige est remarquable en ce que les ailettes d'ancrage sont au nombre de trois et comprennent une ailette d'ancrage latérale et deux ailettes d'ancrage médiales, où les ailettes d'ancrage sont écartées angulairement d'un angle compris entre 110 et 130 degrés, et où l'ailette d'ancrage latérale présente une longueur mesurée radialement supérieure aux longueurs mesurées radialement des ailettes d'ancrage médiales.

Ainsi, grâce à cette conformation, l'ailette d'ancrage latérale est prévue pour s'étendre jusque dans la région latérale vers le trochiter, tandis que les deux ailettes d'ancrage médiales sont prévues pour s'étendre dans la région médiale proche de l'éperon de Merkel à forte densité osseuse. L'éperon de Merkel, appelée également calcar médial, est une région médiale provenant de la corticale, c'est-à-dire de la partie périphérique de l'os, et se prolongeant vers l'intérieur, une telle région médiale ou de Merkel présentant une densité osseuse accrue qui garantit un appui particulièrement stable des deux ailettes d'ancrage médiales et donc une stabilité en torsion accrue. L'ailette d'ancrage latérale a pour vocation de s'étendre dans la région latérale et elle est plus longue ce qui permet d'excentrer le plot d'ancrage et les ailettes d'ancrage médiales pour que ceux-ci s'étendent dans la région (ou zone) médiale à plus forte densité osseuse. En effet, l'ailette d'ancrage latérale, plus longue, va favoriser cet excentrage du composant huméral d'ancrage sans tige en direction de la zone médiale à plus forte densité osseuse, autrement dit en rapprochant le plot d'ancrage et les ailettes d'ancrage médiales vers le côté médial, ce qui permet d'avoir un ancrage du plot d'ancrage et des ailettes d'ancrage médiales dans la zone médiale ou de Merkel.

Selon une caractéristique, les ailettes d'ancrage sont écartées angulairement d'un angle de 120 degrés ± 2 degrés.

Cette répartition angulaire symétrique des ailettes d'ancrage favorise encore la stabilité en torsion.

Selon une variante, le rapport entre la longueur de l'ailette d'ancrage latérale et la longueur d'une ailette d'ancrage médiale est compris entre 1,1 et 1,3, et notamment entre 1,1 et 1,2.

Selon une variante, le plot d'ancrage est un corps de révolution centré sur un axe central, et les longueurs des ailettes d'ancrage sont mesurées radialement à partir de cet axe central, et la longueur de chaque ailette d'ancrage latérale est comprise entre 15 et 25 millimètres, et notamment entre 17 et 21 millimètres, et la longueur d'une ailette d'ancrage médiale est comprise entre 11,5 et 22,5 millimètres, et notamment entre 14,5 et 19 millimètres.

Dans une réalisation particulière, les deux ailettes d'ancrage médiales présentent la même longueur mesurée radialement, pour un appui symétrique équivalent de ces deux ailettes d'ancrage médiales dans la zone médiale.

Avantageusement, les ailettes d'ancrage sont ajourées, pour favoriser une ostéo-intégration ou régénération osseuse à l'intérieur des ajourages des ailettes d'ancrage.

Selon une variante, les ailettes d'ancrage et/ou le plot d'ancrage sont couverts extérieurement et au moins partiellement d'un revêtement de surface métallique poreux ou rugueux favorisant une ostéo-intégration.

Avantageusement, le revêtement métallique poreux ou rugueux est un revêtement bicouche comprenant une couche de titane poreux ou rugueux ou d'un alliage de titane poreux ou rugueux, et une couche de phosphate de calcium, tel que l'hydroxyapatite de calcium.

Dans un mode de réalisation particulier, chaque ailette d'ancrage comprend :
- un segment rectiligne s'étendant radialement à partir d'une partie proximale périphérique du plot d'ancrage ; et
- un segment incurvé reliant une terminaison du segment rectiligne à une partie distale périphérique du plot d'ancrage.

Selon une possibilité de l'invention, la partie proximale périphérique du plot d'ancrage, à partir de laquelle saillent les segments rectilignes des ailettes d'ancrage, est formée d'un bord d'extrémité proximal périphérique du plot d'ancrage.

Ainsi, le bord d'extrémité proximal du plot d'ancrage et les segments rectilignes des ailettes d'ancrage sont coplanaires, pour offrir une liberté accrue pour le chirurgien dans le positionnement du composant huméral d'ancrage sans tige.

Selon une autre possibilité de l'invention, la partie distale périphérique du plot d'ancrage, à partir de laquelle saillent les segments incurvés des ailettes d'ancrage, est formée d'un bord d'extrémité distal périphérique du plot d'ancrage.

Ainsi le bord d'extrémité distal du plot d'ancrage est prolongé radialement par les segments incurvés des ailettes d'ancrage, pour favoriser l'insertion ou enfoncement du composant huméral d'ancrage sans tige.

Conformément à une autre caractéristique avantageuse de l'invention, le plot d'ancrage est creux en étant pourvu d'un orifice interne, pour faciliter la manipulation lors de l'opération d'ancrage et aussi pour faciliter la fixation de l'insert huméral sur le composant huméral d'ancrage sans tige.

Selon une caractéristique, l'orifice interne présente un trou proximal tronconique prolongé par un trou taraudé, optionnellement suivi par un trou distal débouchant, où le trou proximal tronconique sert à la fixation d'un cône morse de l'insert huméral, le trou taraudé sert si nécessaire à l'extraction du composant huméral d'ancrage sans tige au moyen d'un outil d'extraction vissé dans ce trou taraudé.

Selon une autre caractéristique, le plot d'ancrage présente une forme générale tronconique.

La présente invention concerne un implant huméral de prothèse d'épaule, comprenant un composant huméral d'ancrage sans tige selon l'invention, et un insert huméral fixé sur le composant huméral d'ancrage sans tige et présentant :
- soit une calotte hémisphérique conformée pour une articulation avec une glénosphère d'un implant glénoïdien ;
- soit une tête d'articulation sphérique (autrement appelée tête humérale) conformée pour une articulation sur un corps d'articulation d'un implant glénoïdien.

L'invention se rapporte également à une prothèse d'épaule comprenant un implant huméral comme décrit ci-dessus et un implant glénoïdien comprenant :
- soit une glénosphère conformée pour une articulation avec la calotte hémisphérique de l'insert huméral ;
- soit un corps d'articulation conformée pour une articulation avec la tête d'articulation sphérique de l'insert huméral.

L'invention concerne aussi une gamme de composants huméraux d'ancrage sans tige comprenant plusieurs composants huméraux d'ancrage sans tige conformes à l'invention et ayant des tailles distinctes.

D'autres caractéristiques et avantages de la présente invention apparaîtront à la lecture de la description détaillée ci-après, d'un exemple de mise en œuvre non limitatif, faite en référence aux figures annexées dans lesquelles :
- la figure 1 est une vue schématique en perspective d'un composant huméral d'ancrage sans tige selon l'invention ;
- la figure 2 est une vue schématique de dessus du composant huméral d'ancrage sans tige de la figure 1 ;
- les figures 3, 4 et 5 sont des vues schématiques en coupe du composant huméral d'ancrage sans tige des figures 1 et 2 selon les plans de coupe respectivement A-A, B-B et C-C visibles sur la figure 2 ;
- les figures 6 et 7 sont des vues schématiques du composant huméral d'ancrage sans tige des figures 1 et 2 implanté dans un humérus ;
- la figure 8 est une vue schématique en coupe selon le plan de coupe C-C et en superposition de trois composants huméraux d'ancrage sans tige appartenant à une gamme selon l'invention.

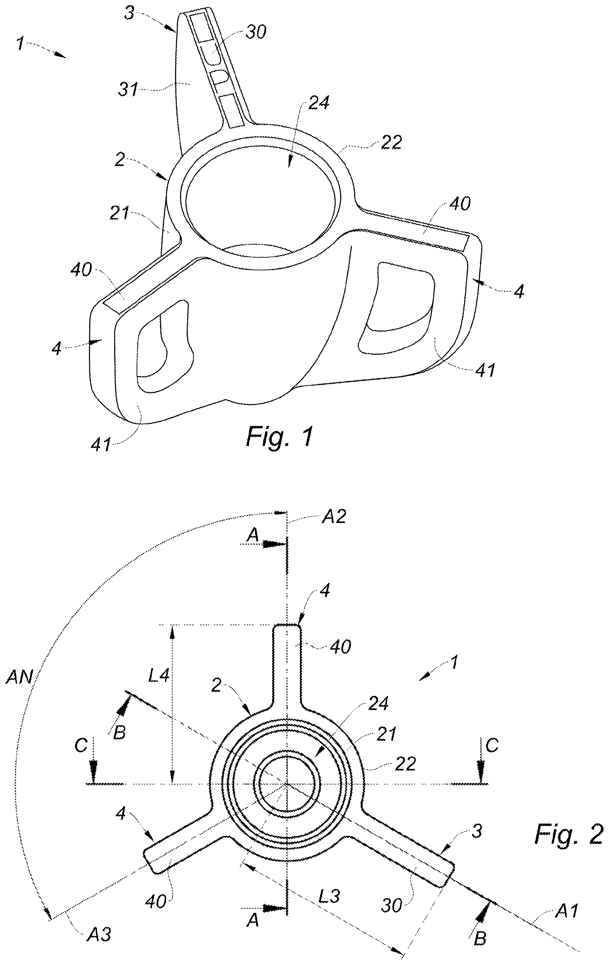

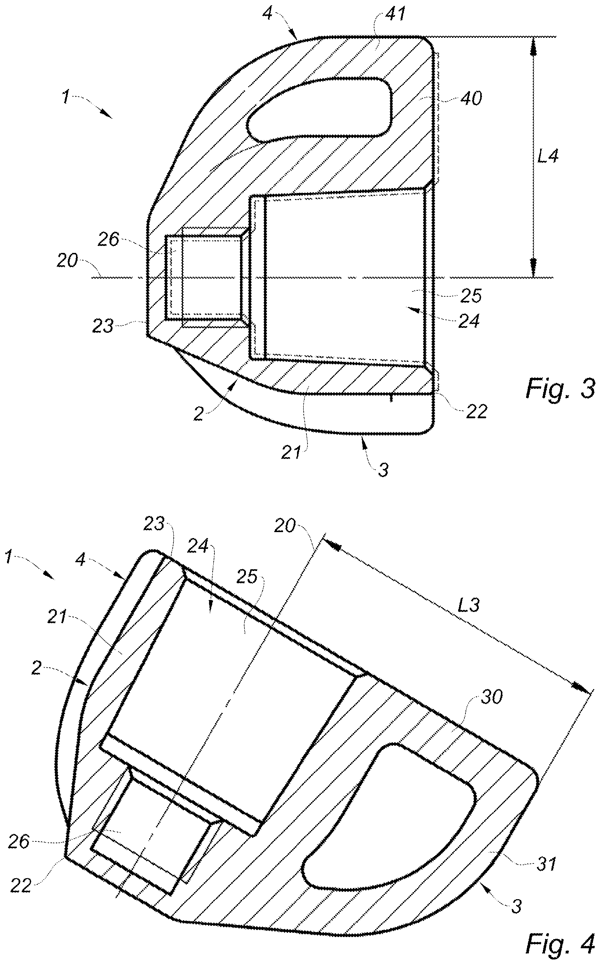

En référence aux figures 1 à 5, un composant huméral d'ancrage sans tige 1 selon l'invention est un composant monobloc réalisé dans un matériau métallique et qui comprend un plot d'ancrage 2 assujetti à trois ailettes d'ancrage 3, 4 s'étendant radialement en périphérie du plot d'ancrage 2.

Le plot d'ancrage 2 est un corps de révolution centré sur un axe central 20 et présentant une paroi périphérique 21 de forme générale tronconique présentant deux bords d'extrémité périphériques opposés :
- un bord d'extrémité proximal périphérique 22 ; et
- un bord d'extrémité distal périphérique 23.

Le plot d'ancrage 2 présente une forme générale tronconique dans le sens d'un rétrécissement en partant du bord d'extrémité proximal périphérique 22 jusqu'au bord d'extrémité distal périphérique 23.

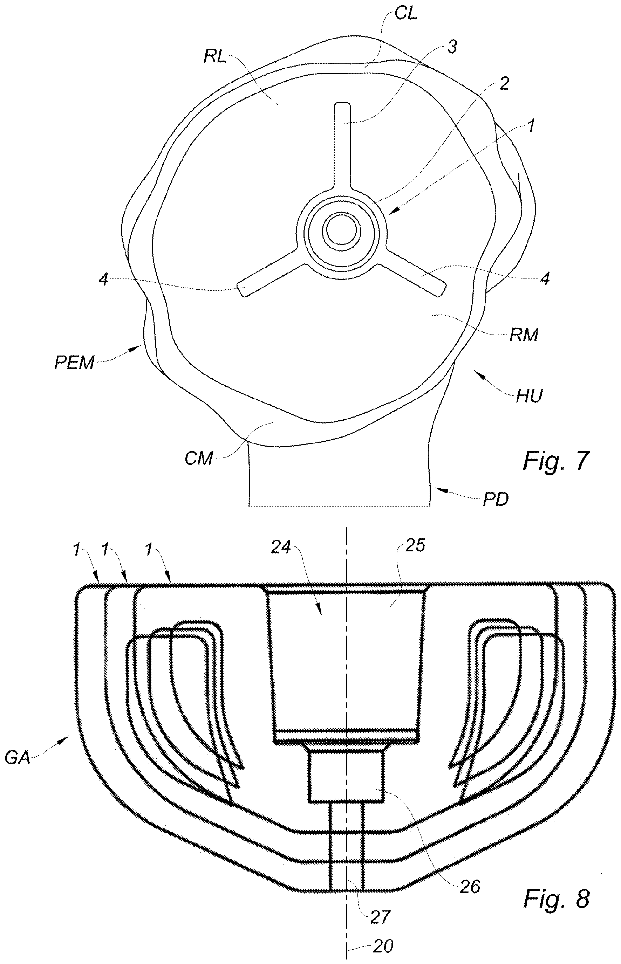

Le plot d'ancrage 2 est creux en étant pourvu d'un orifice interne 24 de révolution centré sur l'axe central 20 et débouchant du côté du bord d'extrémité proximal périphérique 22 et présentant successivement un trou proximal tronconique 25 prolongé par un trou taraudé 26, et optionnellement suivi par un trou distal 27 (visible dans le mode de réalisation de la figure 8) débouchant du côté du bord d'extrémité distal périphérique 23.

Les ailettes d'ancrage 3, 4 s'étendent radialement selon des axes radiaux A1, A2, A3 s'intersectant sur l'axe central 20. Les ailettes d'ancrage 3, 4 font saillie extérieurement de la paroi périphérique 21 du plot d'ancrage 2 et s'étendent dans des plans incluant l'axe central 20 et les axes radiaux A1, A2, A3.

Les ailettes d'ancrage 3, 4 sont écartées angulairement d'un angle AN compris entre 110 et 130 degrés, et de préférence d'un angle AN de 120 degrés ± 2 degrés (c'est-à-dire compris entre 118 et 122 degrés). Autrement dit, chaque ailette d'ancrage 3, 4 est écartée angulairement de chaque autre ailette d'ancrage 3, 4 d'un tel angle AN. Sur la figure 2, seul un angle AN est illustré, sur les trois angles, à des fins de clarté. Les angles AN peuvent être mesurés entre les axes radiaux A1, A2, A3.

Les ailettes d'ancrage 3, 4 comprennent une ailette d'ancrage latérale 3 et deux ailettes d'ancrage médiales 4, où l'ailette d'ancrage latérale 3 présente une longueur L3 mesurée radialement supérieure aux longueurs L4 mesurées radialement des ailettes d'ancrage médiales 4 ; les deux ailettes d'ancrage médiales 4 présentent la même longueur L4 mesurée radialement.

Ces longueurs L3 et L4 sont mesurées en partant de l'axe central 20 selon les axes radiaux A1, A2, A3 respectifs, comme visible sur les figures 2 à 4.

Le rapport entre la longueur L3 de l'ailette d'ancrage latérale 3 et la longueur L4 d'une ailette d'ancrage médiale 4 est compris entre 1,1 et 1,2. La longueur L3 de l'ailette d'ancrage latérale 3 est comprise entre 17 et 21 millimètres, et la longueur L4 d'une ailette d'ancrage médiale 4 est comprise entre 14,5 et 19 millimètres.

Les ailettes d'ancrage 3, 4 sont ajourées, pour favoriser une ostéo-intégration et, à ce titre, chaque ailette d'ancrage 3, 4 comprend :
- un segment rectiligne 30, 40 s'étendant radialement à partir du bord d'extrémité proximal périphérique 22 du plot d'ancrage 2 ; et
- un segment incurvé 31, 41 reliant une terminaison du segment rectiligne 30, 40 au bord d'extrémité distal périphérique 23 du plot d'ancrage 2.

Ainsi, chaque ailette d'ancrage 3, 4 s'étend sur toute la hauteur (distance mesurée le long de l'axe central 20) du plot d'ancrage 2.

Les ailettes d'ancrage 3, 4 et le plot d'ancrage 2 sont couverts extérieurement d'un revêtement de surface métallique poreux ou rugueux favorisant une ostéo-intégration, comme par exemple un revêtement bicouche comprenant une couche de titane poreux ou rugueux ou d'un alliage de titane poreux ou rugueux, et une couche de phosphate de calcium, tel que l'hydroxyapatite de calcium.

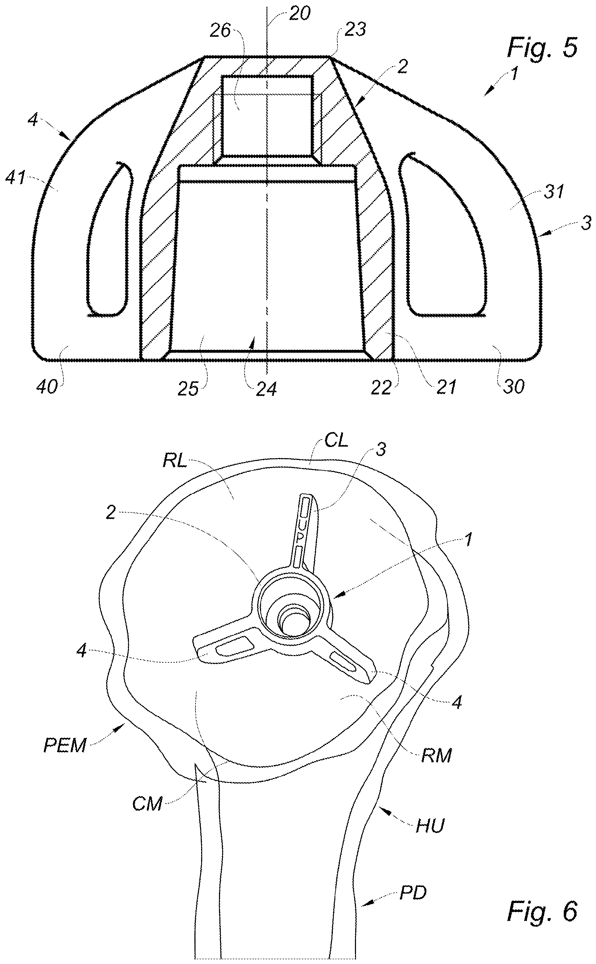

Comme visible sur les figures 6 et 7, le composant huméral d'ancrage sans tige 1 procure un ancrage dans les parties épiphysaire proximal et métaphysaire PEM réséquées de l'humérus HU, sans atteindre la partie diaphysaire PD de l'humérus HU.

Le composant huméral d'ancrage sans tige 1 est positionné de sorte que :
- l'ailette d'ancrage latérale 3 s'étend en direction du côté latéral CL de l'humérus HU, en direction du trochiter jusque dans la région latérale RL à densité osseuse moindre comparativement à la région médiale RM,
- les deux ailettes d'ancrage médiales 4 sont tournées vers le côté médial CM de l'humérus HU pour s'étendre dans la région médiale RM de l'éperon de Merkel à forte densité osseuse.

Plus précisément, le composant huméral d'ancrage sans tige 1 est positionné de manière excentrée, dans le sens où ce dernier est rapproché vers le côté médial CM principalement du fait de la longueur accrue de l'ailette d'ancrage latérale 3 qui favorise cet excentrage, et donc en rapprochant le plot d'ancrage 2 et les ailettes d'ancrage médiales 4 vers le côté médial CM, ce qui permet d'avoir les ailettes d'ancrage médiales 4 qui s'étendent quasiment intégralement dans la région médiale RM appelée également calcar médial procurant ainsi un appui majoritairement dans la région médiale RM à forte densité osseuse, tandis que l'ailette d'ancrage latérale 3 s'étend quasiment intégralement dans la région latérale RL d'os spongieux.

Pour faciliter le guidage du composant huméral d'ancrage sans tige 1 jusqu'à sa position définitive d'implantation, telle qu'illustré sur les figures 6 et 7, le chirurgien peut employer une broche de guidage qui coopère avec l'orifice interne 24, de sorte que le chirurgien commence par positionner la broche de guidage et fait glisser ensuite le plot d'ancrage 2 sur cette broche de guidage jusqu'à la position d'implantation définitive, et la broche de guidage est ensuite enlevée. L'utilisation d'une broche de guidage laisse la possibilité de régler la bonne orientation des ailettes d'ancrage 3, 4, en orientant l'ailette d'ancrage latérale 3 en direction du côté latéral CL, autrement dit en direction du trochiter.

Ainsi, grâce à cette conformation du composant huméral d'ancrage sans tige 1 et grâce à cette implantation, il est garantit un appui particulièrement stable des ailettes d'ancrage médiales 4 dans la région médiale RM à densité osseuse accrue, et donc une stabilité globale accrue pour le composant huméral d'ancrage sans tige 1.

Comme visible sur la figure 8, ce composant huméral d'ancrage sans tige 1 peut être décliné au sein d'une gamme GA comprenant plusieurs composants huméraux d'ancrage sans tige 1 ayant des tailles distinctes, et notamment ayant des hauteurs (dimensions mesurées le long de l'axe central), des longueurs (dimensions mesurées radialement) et des épaisseurs pour les segments des ailettes d'ancrage qui croissent avec la taille. Cependant, les dimensions de l'orifice interne 24 restent identiques d'une taille à l'autre.

Bien entendu l'exemple de mise en œuvre évoqué ci-dessus ne présente aucun caractère limitatif et d'autres améliorations et détails peuvent être apportés au composant huméral d'ancrage sans tige selon l'invention, sans pour autant sortir du cadre de l'invention où d'autres types de revêtement poreux ou rugueux peuvent par exemple être envisagés, ou bien d'autres formes d'orifice interne peuvent être prévues.

## Revendications

1. Composant huméral d'ancrage sans tige (1) pour implant huméral de prothèse d'épaule, ledit composant huméral d'ancrage sans tige (1) comportant un plot d'ancrage (2) assujetti à des ailettes d'ancrage (3, 4) s'étendant radialement en périphérie du plot d'ancrage (2), **caractérisé en ce que** les ailettes d'ancrage (3, 4) sont au nombre de trois et comprennent une ailette d'ancrage latérale (3) et deux ailettes d'ancrage médiales (4), où les ailettes d'ancrage (3, 4) sont écartées angulairement d'un angle (AN) compris entre 110 et 130 degrés, et où l'ailette d'ancrage latérale (3) présente une longueur (L3) mesurée radialement supérieure aux longueurs (L4) mesurées radialement des ailettes d'ancrage médiales (4).

2. Composant huméral d'ancrage sans tige (1) selon la revendication 1, dans lequel les deux ailettes d'ancrage médiales (4) présentent la même longueur (L4) mesurée radialement.

3. Composant huméral d'ancrage sans tige (1) selon l'une quelconque des revendications 1 et 2, dans lequel le rapport entre la longueur (L3) de l'ailette d'ancrage latérale (3) et la longueur (L4) d'une ailette d'ancrage médiale (4) est compris entre 1,1 et 1,3.

4. Composant huméral d'ancrage sans tige (1) selon la revendication précédente, dans lequel le plot d'ancrage (2) est un corps de révolution centré sur un axe central (20), et les longueurs (L3, L4) des ailettes d'ancrage (3, 4) sont mesurées radialement à partir de cet axe central (20), et la longueur (L3) de l'ailette d'ancrage latérale (3) est comprise entre 15 et 25 millimètres, et notamment entre 17 et 21 millimètres, et la longueur (L4) de chaque ailette d'ancrage médiale (4) est comprise entre 11,5 et 22,5 millimètres, et notamment entre 14,5 et 19 millimètres.

5. Composant huméral d'ancrage sans tige (1) selon l'une quelconque des revendications précédentes, dans lequel les ailettes d'ancrage (3, 4) sont écartées angulairement d'un angle (AN) de 120 degrés ± 2 degrés.

6. Composant huméral d'ancrage sans tige (1) selon l'une quelconque des revendications précédentes, dans lequel les ailettes d'ancrage (3, 4) sont ajourées.

7. Composant huméral d'ancrage sans tige (1) selon l'une quelconque des revendications précédentes, dans lequel chaque ailette d'ancrage (3 ; 4) comprend :
- un segment rectiligne (30 ; 40) s'étendant radialement à partir d'une partie proximale périphérique (22) du plot d'ancrage (2) ; et
- un segment incurvé (31 ; 41) reliant une terminaison du segment rectiligne (30 ; 40) à une partie distale périphérique (23) du plot d'ancrage (2).

8. Composant huméral d'ancrage sans tige (1) selon la revendication précédente, dans lequel la partie proximale périphérique du plot d'ancrage (2), à partir de laquelle saillent les segments rectilignes (30, 40) des ailettes d'ancrage (3, 4), est formée d'un bord d'extrémité proximal périphérique (22) du plot d'ancrage (2).

9. Composant huméral d'ancrage sans tige (1) selon l'une quelconque des revendications 7 et 8, dans lequel la partie distale périphérique du plot d'ancrage (2), à partir de laquelle saillent les segments incurvés (21, 31) des ailettes d'ancrage (3, 4), est formée d'un bord d'extrémité distal périphérique (23) du plot d'ancrage (2).

10. Composant huméral d'ancrage sans tige (1) selon l'une quelconque des revendications précédentes, dans lequel le plot d'ancrage (2) est creux en étant pourvu d'un orifice interne (24).

11. Composant huméral d'ancrage sans tige (1) selon la revendication précédente, dans lequel l'orifice interne (24) présente un trou proximal tronconique (25) prolongé par un trou taraudé (26).

12. Composant huméral d'ancrage sans tige (1) selon l'une quelconque des revendications précédentes, dans lequel le plot d'ancrage (2) présente une forme générale tronconique.

13. Implant huméral de prothèse d'épaule, comprenant un composant huméral d'ancrage sans tige (1) selon l'une quelconque des revendications précédentes, et un insert huméral fixé sur le composant huméral d'ancrage sans tige (1) et présentant :
- soit une calotte hémisphérique conformée pour une articulation avec une glénosphère d'un implant glénoïdien ;
- soit une tête d'articulation sphérique conformée pour une articulation sur un corps d'articulation d'un implant glénoïdien.

14. Prothèse d'épaule comprenant un implant huméral selon la revendication 13, et un implant glénoïdien comprenant :
- soit une glénosphère conformée pour une articulation avec la calotte hémisphérique de l'insert huméral ;
- soit un corps d'articulation conformée pour une articulation avec la tête d'articulation sphérique de l'insert huméral.

15. Gamme (GA) de composants huméraux d'ancrage sans tige (1) comprenant plusieurs composants huméraux d'ancrage sans tige (1) conformes à l'une quelconque des revendications 1 à 12 et ayant des tailles distinctes.

## Patentansprüche

1. Komponente für die humerale Verankerung ohne Schaft (1) für ein humerales Schulterprothesenimplantat, wobei die Komponente für die humerale Verankerung ohne Schaft (1) ein Verankerungsstück (2) aufweist, das mit Verankerungsflügeln (3, 4) belegt ist, die sich radial an der Peripherie des Verankerungsstücks (2) erstrecken, **dadurch gekennzeichnet, dass** die Verankerungsflügel (3, 4) in der Anzahl von drei vorliegen und einen seitlichen Verankerungsflügel (3) und zwei mediale Verankerungsflügel (4) umfassen, wobei die Verankerungsflügel (3, 4) winklig in einem Winkel (AN) zwischen 110 und 130 Grad beabstandet sind und wobei der seitliche Verankerungsflügel (3) eine radial gemessene Länge (L3) aufweist, die größer als die radial gemessenen Längen (L4) der medialen Verankerungsflügel (4) ist.

2. Komponente für die humerale Verankerung ohne Schaft (1) nach Anspruch 1, wobei die zwei medialen Verankerungsflügel (4) dieselbe radial gemessene Länge (L4) aufweisen.

3. Komponente für die humerale Verankerung ohne Schaft (1) nach einem der Ansprüche 1 und 2, wobei das Verhältnis zwischen der Länge (L3) des seitlichen Verankerungsflügels (3) und der Länge (L4) eines medialen Verankerungsflügels (4) zwischen 1,1 und 1,3 liegt.

4. Komponente für die humerale Verankerung ohne Schaft (1) nach vorangehendem Anspruch, wobei das Verankerungsstück (2) ein auf einer zentralen Achse (20) zentrierter Drehkörper ist und die Längen (L3, L4) der Verankerungsflügel (3, 4) radial ab dieser zentralen Achse (20) gemessen werden und die Länge (L3) des seitlichen Verankerungsflügels (3) zwischen 15 und 25 Millimeter und insbesondere zwischen 17 und 21 Millimeter liegt und die Länge (L4) jedes medialen Verankerungsflügels (4) zwischen 11,5 und 22,5 Millimeter und insbesondere zwischen 14,5 und 19 Millimeter liegt.

5. Komponente für die humerale Verankerung ohne Schaft (1) nach einem der vorangehenden Ansprüche, wobei die Verankerungsflügel (3, 4) winklig in einem Winkel (AN) von 120 Grad ± 2 Grad beabstandet sind.

6. Komponente für die humerale Verankerung ohne Schaft (1) nach einem der vorangehenden Ansprüche, wobei die Verankerungsflügel (3, 4) durchbrochen sind.

7. Komponente für die humerale Verankerung ohne Schaft (1) nach einem der vorangehenden Ansprüche, wobei jeder Verankerungsflügel (3; 4) umfasst:
- ein gerades Segment (30; 40), das sich radial ab einem peripheren proximalen Teil (22) des Verankerungsstücks (2) erstreckt; und
- ein gekrümmtes Segment (31; 41), das ein Ende des geraden Segments (30; 40) mit einem peripheren distalen Teil (23) des Verankerungsstücks (2) verbindet.

8. Komponente für die humerale Verankerung ohne Schaft (1) nach vorangehendem Anspruch, wobei der periphere proximale Teil des Verankerungsstücks (2), ab dem die geraden Segmente (30, 40) der Verankerungsflügel (3, 4) hervorstehen, von einem peripheren proximalen Endrand (22) des Verankerungsstücks (2) gebildet ist.

9. Komponente für die humerale Verankerung ohne Schaft (1) nach einem der Ansprüche 7 und 8, wobei der periphere distale Teil des Verankerungsstücks (2), ab dem die gekrümmten Segmente (21, 31) der Verankerungsflügel (3, 4) hervorstehen, von einem peripheren distalen Endrand (23) des Verankerungsstücks (2) gebildet ist.

10. Komponente für die humerale Verankerung ohne Schaft (1) nach einem der vorangehenden Ansprüche, wobei das Verankerungsstück (2) hohl ist, indem es mit einer inneren Öffnung (24) ausgestattet ist.

11. Komponente für die humerale Verankerung ohne Schaft (1) nach vorangehendem Anspruch, wobei die innere Öffnung (24) ein kegelstumpfförmiges proximales Loch (25) aufweist, das von einem Gewindeloch (26) verlängert wird.

12. Komponente für die humerale Verankerung ohne Schaft (1) nach einem der vorangehenden Ansprüche, wobei das Verankerungsstück (2) eine allgemeine Kegelstumpfform aufweist.

13. Humerales Schulterprothesenimplantat, umfassend eine Komponente für die humerale Verankerung ohne Schaft (1) nach einem der vorangehenden Ansprüche und einen humeralen Einsatz, der auf der Komponente für die humerale Verankerung ohne Schaft (1) befestigt ist, und aufweisend:
- entweder eine halbkugelige Kappe, die für ein Gelenk mit einer Kugelpfanne eines Kugelpfannenimplantats ausgebildet ist;
- oder einen kugeligen Gelenkkopf, der für ein Gelenk auf einem Gelenkkörper eines Kugelpfannenimplantats ausgebildet ist.

14. Schulterprothese, umfassend ein humerales Implantat nach Anspruch 13 und ein Kugelpfannenimplantat, umfassend:
- entweder eine Kugelpfanne, die für ein Gelenk mit der halbkugeligen Kappe des humeralen Implantats ausgebildet ist;
- oder einen Gelenkkörper, der für ein Gelenk mit dem kugeligen Gelenkkopf des humeralen Implantats ausgebildet ist.

15. Sortiment (GA) aus Komponenten für die humerale Verankerung ohne Schaft (1), umfassend mehrere Komponenten für die humerale Verankerung ohne Schaft (1), die nach einem der Ansprüche 1 bis 12 ausgebildet sind und unterschiedliche Größen haben.

## Claims

1. A stemless humeral anchoring component (1) for a shoulder prosthesis humeral implant, said stemless humeral anchoring component (1) including an anchoring stud (2) secured to anchoring wings (3, 4) extending radially along the periphery of the anchoring stud (2), **characterized in** the anchoring wings (3, 4) are three in number and comprise one lateral anchoring wing (3) and two medial anchoring wings (4), wherein the anchoring wings (3, 4) are angularly spaced by an angle (AN) comprised between 110 and 130 degrees, and wherein the lateral anchoring wing (3) has a radially measured length (L3) greater than the radially measured lengths (L4) of the medial anchoring wings (4).

2. The stemless humeral anchoring component (1) according to claim 1, wherein the two medial anchoring wings (4) have the same radially measured length (L4).

3. The stemless humeral anchoring component (1) according to any one of claims 1 and 2, wherein the ratio between the length (L3) of the lateral anchoring wing (3) and the length (L4) of a medial anchoring wing (4) is comprised between 1.1 and 1.3.

4. The stemless humeral anchoring component (1) according to the preceding claim, wherein the anchoring stud (2) is a body of revolution centered on a central axis (20), and the lengths (L3, L4) of the anchoring wings (3, 4) are measured radially from this central axis (20), and the length (L3) of the lateral anchoring wing (3) is comprised between 15 and 25 millimeters, and in particular between 17 and 21 millimeters, and the length (L4) of each medial anchoring wing (4) is comprised between 11.5 and 22.5 millimeters, and in particular between 14.5 and 19 millimeters.

5. The stemless humeral anchoring component (1) according to any one of the preceding claims, wherein the anchoring wings (3, 4) are angularly spaced by an angle (AN) of 120 degrees ± 2 degrees.

6. The stemless humeral anchoring component (1) according to any one of the preceding claims, wherein the anchoring wings (3, 4) are perforated.

7. The stemless humeral anchoring component (1) according to any one of the preceding claims, wherein each anchoring wing (3; 4) comprises:
- a rectilinear segment (30; 40) extending radially from a peripheral proximal portion (22) of the anchoring stud (2); and
- a curved segment (31; 41) connecting a termination of the rectilinear segment (30; 40) to a peripheral distal portion (23) of the anchoring stud (2).

8. The stemless humeral anchoring component (1) according to the preceding claim, wherein the peripheral proximal portion of the anchoring stud (2), from which the rectilinear segments (30, 40) of the anchoring wings (3, 4) protrude, is formed of a peripheral proximal end edge (22) of the anchoring stud (2).

9. The stemless humeral anchoring component (1) according to any one of claims 7 and 8, wherein the peripheral distal portion of the anchoring stud (2), from which the curved segments (21, 31) of the anchoring wings (3, 4) protrude, is formed of a peripheral distal end edge (23) of the anchoring stud (2).

10. The stemless humeral anchoring component (1) according to any one of the preceding claims, wherein the anchoring stud (2) is hollow by being provided with an inner orifice (24).

11. The stemless humeral anchoring component (1) according to the preceding claim, wherein the inner orifice (24) has a frustoconical proximal hole (25) extended by a tapped hole (26).

12. The stemless humeral anchoring component (1) according to any one of the preceding claims, wherein the anchoring stud (2) has a generally frustoconical shape.

13. A shoulder prosthesis humeral implant, comprising a stemless humeral anchoring component (1) according to any one of the preceding claims, and a humeral insert fastened on the stemless humeral anchoring component (1) and having :
- either a hemispherical cap shaped for a joint with a glenosphere of a glenoid implant;
- or a spherical joint head shaped for a joint on a joint body of a glenoid implant.

14. A shoulder prosthesis comprising a humeral implant according to claim 13, and a glenoid implant comprising:
- either a glenosphere shaped for a joint with the hemispherical cap of the humeral insert;
- or a joint body shaped for a joint with the spherical joint head of the humeral insert.

15. A range (GA) of stemless humeral anchoring components (1) comprising several stemless humeral anchoring components (1) according to any one of claims 1 to 12 and having distinct sizes.
